# EUROPEAN PATENT APPLICATION

(11) **EP 1 209 154 A1**
(43) Date of publication of application: **29.05.2002**
(21) Application number: 00125084.4
(22) Date of filing: 17.11.2000
(51) Int. Cl.: C07D 277/74, C07C 61/40, C07C 43/23, C07C 205/11, C07C 21/18, C07C 21/215, C07C 22/00, C07C 22/08

(54) **Preparation of monofluoroalkenes**

(71) Applicant: Aventis CropScience GmbH, 65929 Frankfurt am Main (DE)
(72) Inventor: Pazenok, Sergiy, Dr., 65779 Kelkheim (DE); Demoute, Jean Pierre, 93360 Neuilly Plaisance (FR); Zard, Samir, 91190 Gif-Sur-Yvette (FR); Lequeux, Thierry, 14800 Langrune Sur Mer (FR)

(57) **Abstract**

There is described a process for the preparation of compounds of formula I 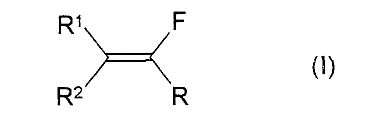 in which
R is hydrogen, optionally substituted alkyl, optionally substituted alkenyl optionally substituted cycloalkyl, optionally substituted phenyl or optionally substituted heterocyclyl, and
R¹ and R² are hydrogen, an organic radical or together with the ring to which they are attached, form an optionally substituted ring, which may contain one or more hetero atoms,
which comprises reacting a compound of formula II 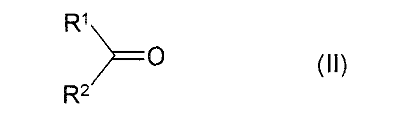 with a compound of formula III 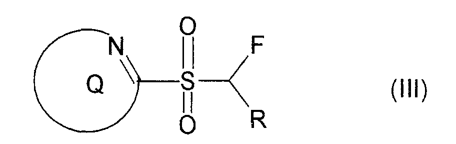 in which the ring Q is an optionally substituted heteroaromatic ring, which optionally comprises one or more further hetero atoms and to which is optionally fused an optionally substituted ring.

## Description

This invention relates to a process for preparing compounds containing a monofluorovinyl group.

The introduction of a fluorovinyl moiety into aldehydes or ketones can be used for the preparation of fluoroalkenes and derivatives thereof. There are several disclosures of reagents that can be used for the introduction of this moiety onto aldehydes.

Fluoromethylenetriphenylphosphorane, Ph₃P=CHF, can function for the introduction of a fluorovinyl moiety into organic compounds (see J. Org. Chem. **1975**, *40*, 2796-2801). The major limitation for this reagent is that the ylide could only be obtained from poorly accessible fluoroiodomethane prepared in yield of approximately 20% from the reaction of mercuric fluoride and methylene iodide.

The use of phenylsulfonylfluoromethane, PhSO₂CH₂F, (see JACS, **92**, *114*, 360-361) for the olefination comprises the reaction of phenylsulfonylfluoromethyllithium (generated by the reaction of phenylsulfonylfluoromethane with butyllithium) with carbonyl compounds to form α-fluoro-β-hydroxyphenylsulfonyl derivatives, dehydration with methanesulfonyl chloride to the corresponding vinyl sulfones, followed by reductive desulfonylation with aluminium amalgam. The same complex and multi steps procedure has been used for the olefination with 1-fluoroethylsulfinylbenzene, PhSOCHFCH₃,(Tetrahedron Lett. **1992**, 33, 1483-1484).

In Tetrahedron Lett. (1988), 29 (22) p.2655-2658 is described the use of Horner-Emmons reagents, such as for the introduction of a =CF-COOEt fragment. Further transformation into a 1-fluoroethylidene, =C(F)(Me), group is described in WO 99/32426. A large number of steps are required to achieve the desired products.

In Bull Soc Chim Fr **1993**, 856-878, is disclosed the reaction of 2-chloromethysulfonylbenzothiazole with nonanal and also with 4-methoxybenzaldehyde to replace the oxygen of the aldehyde group with a chloromethylene group. The reaction is carried out using a strong base (lithium diisopropylamide) and thus proceeds via the formation of a carbanion. An α-chlorine is known to stabilize a carbanion much better then an α-fluorine. That is a main reason why the acidity of haloforms decreases in the order Br₃CH>Cl₃CH>F₃CH so that the corresponding anions appear to increase in stability in series CF₃<CCl₃<CBr₃ (W. Sheppard, C. Shafts, Organic Fluorine Chemistry, 1969, p.26). Therefore one would expect that a similar reaction with fluorine instead of chlorine would only proceed with great difficulty, if at all. We have now surprisingly found that fluorosulfones of Formula III (see later) react very easily with aldehydes and ketones, and the reaction does not require harsh conditions to give monofluoroalkenes in a high yield and good purity.

The invention thus provides a process for the preparation of compounds of formula I 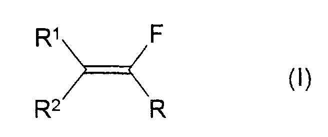 in which
R is hydrogen, optionally substituted alkyl, optionally substituted alkenyl optionally substituted cycloalkyl, optionally substituted phenyl or optionally substituted heterocyclyl, and
R¹ and R², which may be the same or different, are hydrogen, an organic radical or together with the ring to which they are attached, form an optionally substituted ring, which may contain one or more hetero atoms,
which comprises reacting a compound of formula II 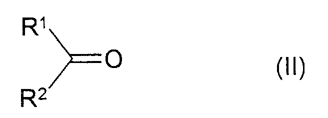 with a compound of formula III 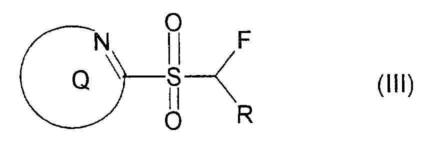 in which the ring Q is an optionally substituted heteroaromatic ring, which optionally comprises one or more further hetero atoms and to which is optionally fused an optionally substituted ring.

R is preferably hydrogen, optionally substituted C₁₋₅-alkyl, especially methyl, cycloalkyl, haloalkyl, especially perfluoroalkyl, C₂₋₅-alkenyl, e.g. allyl, optionally substituted phenyl or optionally substituted heteroaryl.

Examples of radicals R¹ and R² are identical or different alkyl, alkenyl, alkynyl, cycloalkyl, phenyl each of can be optionally substituted, e.g. by phenyl (optionally substituted, e.g. by halogen, phenyl, alkyl, alkoxy, haloalkyl or phenoxy), cyano, halogen, hydroxy, alkoxy, carboxy or alkoxycarbonyl.

Optional substituents on any phenyl or heterocyclyl group include halogen, optionally substituted phenyl, alkyl, alkoxy, haloalkyl or phenoxy). Cycloalkyl and phenyl may also be optionally substituted by optionally substituted alkyl, e.g. haloalkyl and vice versa.

Any alkyl group is preferably of 1 to 12 carbon atoms, especially of 1 to 7 carbon atoms, and particularly of 1 to 5 carbon atoms.

Any alkenyl or alkynyl group is preferably of 2 to 7 carbon atoms, for example allyl, vinyl or propargyl.

Any cycloalkyl is preferably of 3 to 7 carbon atoms, and especially cyclopropyl.

The term heterocyclyl, unless otherwise defined, means 5, 6 or 7-membered rings containing up to 4 hetero atoms selected from nitrogen, oxygen and sulfur. Preferably the heterocyclyl is aromatic, i.e. a heteroaryl group. Examples include furyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyrimidinyl, triazinyl, benzimidazolyl, tetrazolyl, benzoxazolyl and benzofuranyl, benzothiazolyl.

### R¹ is preferably hydrogen

A particularly preferred group for R² is 3-carboxy-2,2-dimethylcyclopropyl. The resultant compound of formula (II) is a precursor for the preparation of pyrethroid insecticides claimed in WO 99/32426 and then invention includes these compounds when obtained from the precursor obtained by the process of the invention. These compounds are of formula X 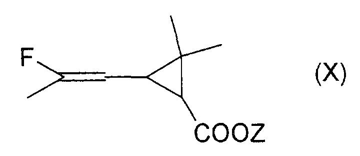 where Z is the residue of a pyrethroid alcohol.

The pyrethroids are thus obtained by further reacting the compound of formula I, obtained by the process of the invention, in which R is methyl, R¹ is hydrogen and R² is 3-carboxy-2,2-dimethylcyclopropyl or a functional derivative of the compound of formula I, especially the acid chloride, is reacted with an alcohol, ZOH, or a functional derivative thereof. Further details of carrying out this conversion are disclosed in WO 99/32426.

Examples of heteroaromatic rings, Q, are 2-imidazolyl, 2-pyridyl, 2-pyrimidyl, 1-phenyl-1H-tetrazol-5-yl, 2-benzothiazolyl , which is especially preferred, 2-benzimidazolyl, 5-alkyl-2-benzothiazolyl, 6-(chloro-or bromo)-2-benzothiazolyl.

The reaction is generally carried out under basic conditions, preferably a metalo organic compound, such as lithium diisopropylamide, butyllithium, sodium or potassium bis(trimethylsilyl)amide, sodium tert.-amylate or potassium tert:-butylate, the last being preferred.

The reaction is usually carried out a temperature of between -100°C and 25°C, suitable solvents for instance are tetrahydrofuran, diethyl or diisopropyl ether, dimethoxyethane, diglyme, hydrocarbons, such as toluene, hexane, pentane or cyclohexane, chlorinated hydrocarbons such as methylene chloride, dimethyl formamide or N-methylpyrrolidone. The reaction usually gives a cis/trans isomer mixture. However, in a simple manner, known to the person skilled in the art, it is possible to influence the isomer ratio by reaction conditions (time, temperature, solvent) or by the addition of different salts.

The compounds of formula III, which are novel, can be prepared by oxidation, in known manner, of a compound of formula IV

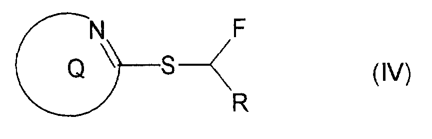

Suitable oxidising reagents include oxone, m-chloroperbenzoic acid, hydrogen peroxide or peracetic acid.. The reaction is usually carried out in a solvent, e.g. a chlorinated hydrocarbon, such as methylene dichloride, aqueous methanol or acetic acid at a temperature usually between room temperature and reflux.

The compounds of formula IV can be prepared according the several methods:
One method is by fluoroalkylation of a compound of formula V 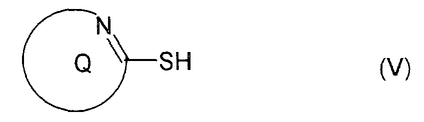 with a compound of formula VI 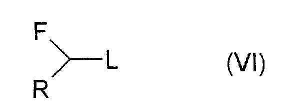 where L is leaving group, such as Cl or Br, usually in the presence of base, such as an alkali metal hydroxide, hydride, alkoxide or amide, optionally in the presence of a solvent. The compounds of formula V or VI are generally known or can be obtained in known manner.

More usually the compounds of formula IV are obtained by nucleophilic fluorination of a compound of formula VII 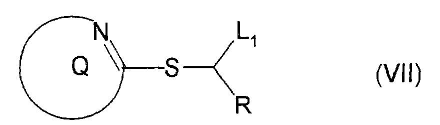 where L₁ is a leaving group, such as chlorine (which is preferred), bromine, iodine or a sulfonate group, e.g. toluenesulfonate.

Suitable fluorinating agents include alkali metal fluorides such as sodium, potassium and caesium fluoride, tetralkylammonium fluorides, such as Me₄NF, Bu₄NF and Bu₄NH₂F₃, NEt₃.3HF, hydrogen fluoride and pyridine.HF, optionally in the presence of 10 to 200% by weight of the compound of formula VII of a Lewis acid, such as a halide (especially a fluoride, bromide or chloride) of boron, antimony, zinc, titanium, or tin. The fluorinating reagent is usually used in an amount of 0.3 to 20, preferably 0.5 to 5, equivalents per equivalent of the compound of formula VII. The reaction is usually carried out at a temperature of -10°C to 150°C.

It is possible to prepare a compound of formula III by electrophilic fluorination of the corresponding sulfones of the formula IIIa

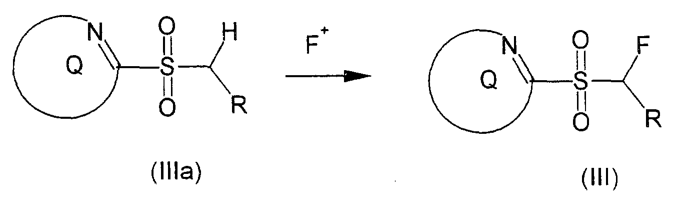

Suitable electrophilic reagents are fluorine, N-F reagents, such as "Selectfluor" (F-TEDA-BF₄ or 1-chloromethyl-4-fluoro-1,4-diazoniabicyclo[2.2.2]octane bis(tetrafluoroborate), Accufluor or (1-hydroxy-4-fluoro-1,4-diazoniabicyclo[2.2.2]octane bis(tetrafluoroborate, N-fluoropyridinium salts or N-fluorobis(phenylsulfonyl)amine.

Suitable solvents include dimethylformamide, acetonitrile, ethers, such as tetrahydrofuran or diethyl ether, acetic acid, trifluoroacetic acid, chlorinated hydrocarbons, such as dichloromethane or dichloroethane, and fluorocarbons.

It is also possible to obtain the compound of formula III via fluorination of the corresponding sulfides (formula VIII) or sulfoxides (formula VIIIa) with DAST-(dialkylamino)sulfurtrifluoride)

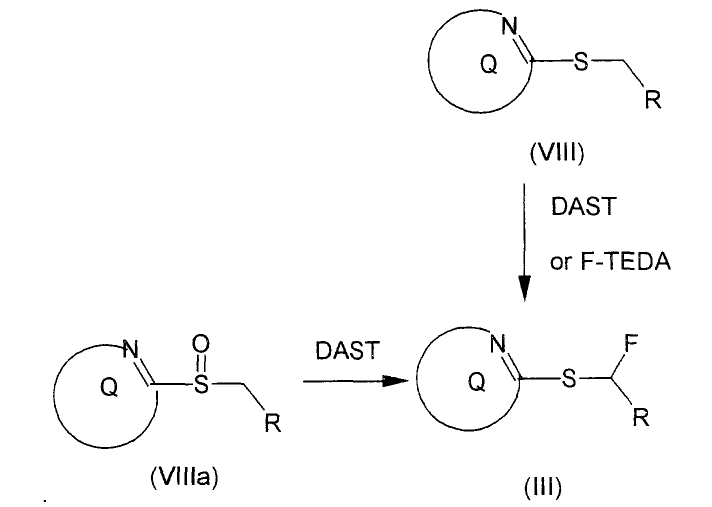

The compound of formula VII can be obtained by introducing the group L₁ in known manner into a compound of formula VIII

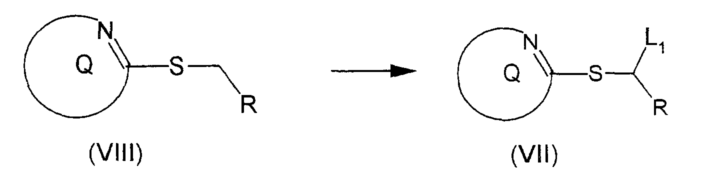

Where L₁ is a halogen, this can be done using a suitable halogenating agent, such a N-halosuccinimide.

The compounds of formula VIII are known or can be obtained in known manner from the appropriate mercapto compound.

The compounds of formula Villa can be obtained by oxidising in known manner the compounds of formula VIII.

The invention is illustrated in the following examples

### Example 1

### 2-Ethylthiobenzothiazole

In a three necked flask containing 2-mercaptobenzothiazole (30 g), bromoethane (21.8 g), and tetrabutylammonium bromide (2 g) in toluene (1000 ml) a solution of sodium hydroxide (150 g) in water (300 ml) was added. The mixture was vigorously stirred at room temperature for 5 hours. The solution was cooled, washed with brine, dried over magnesium sulfate and the solvent removed under reduced pressure to give the title product as yellow crystals, m.p. 27-28°C, in 94% yield.

### Example 2

### 2-(1-Chloroethylthio)benzothiazole

A solution of 2-ethylthiobenzothiazole (20 g) in methylene chloride (500 ml) was heated to 50°C and then N-chlorosuccinimide (20.5g) was added in one portion. The mixture was stirred for 2 hours at 50°C. The mixture was washed with water, dried over magnesium sulfate and the solvent removed under reduced pressure to give the title product, as a brown oil, in 100% yield. H¹ NMR ( δ ppm): 2,06 (d, ³*J*_{HH}=6,751 Hz, 3H, CH₃) ; 6,15 (q, ³*J*_{HH}=6,692 Hz, 1H, CHCl) ; 7,38 (t, ³*J*_{HH}=7,509 Hz, 1H, CH aro) ; 7,49 (t, ³*J*_{HH}=7,125 Hz, 1H, CH aro) ; 7,83 (d, ³*J*_{HH}=7,959 Hz, 1H, CH aro); 8,00 (d, ³*J*_{HH}=8,151 Hz, 1H, CH aro).

### Example 3

### a) 2-(1-Fluoroethylthio)benzothiazole by nuclephilic fluorination

A mixture of 2-(1-chloroethylthio)benzothiazole (22.9 g), anhydrous zinc bromide (11.25 g) and triethylamine trihydrofluoride (64.4 g) was stirred at 70°C for 6 hours. The mixture was cooled, dissolved in ethyl acetate (400 ml), washed with water and dried over magnesium sulfate. The solvent was removed in vacuum to give the crude title product (91% purity, 78% yield) which was used without purification.
H¹ NMR( δ ppm): 1,88 (dd, ³*J*_{HH}=6,47 Hz, ³*J*_{HF}=20,29 Hz, 3H, CH₃) ; 6,71 (dq, ³*J*_{HH}=6,448 Hz, ²*J*_{HF}=53,85 Hz, 1H, CHF) ; 7,38 (t, ³*J*_{HH}=7,486 Hz, 1H, CH aro); 7,48 (t, ³*J*_{HH}=7,46 Hz, 1H, CH aro) ; 7,83 (d, ³*J*_{HH}=7,96 Hz, 1H, CH aro) ; 7,99 (d, ³*J*_{HH}=8,126 Hz, 1H, CH aro).
¹⁹F NMR ( δ ppm):-142,4 (dq, ³*J*_{HF}=21,17 Hz, ²*J*_{HF}=54,12 Hz, 1F, CHF).

### b) 2-(1-Fluoroethylthio)benzothiazole using bromofluoroethane

A mixture of 2-mercaptobenzothiazole (4.2 g) of potassium hydroxide (1.42 g), tetrabutylammonium bromide (0.5 g) and (20 ml) of 1-bromo-1-fluoroethane was stirred for 4 hours at room temperature. Excess 1-bromo-1-fluoroethane was distilled off, the residue was dissolved in methylene chloride (20 ml), washed with water and dried over magnesium sulfate. The solvent was removed under reduced pressure to give the crude title product (4.1 g) which was used without purification.

### Example 4

### 2-(1-Fluoroethylsulfonyl)benzothiazole

To a solution of 2-(1-fluoroethylthio)benzothiazole (21.3 g) in methylene chloride was added m-chloroperbenzoic acid (50 g), portionwise. The mixture was stirred overnight, at room temperature, and then washed with 1N sodium hydroxide solution. The solvent was removed under reduced pressure and the crude product was recrystallized from methanol to give the title product; m.p. 75-78°C, in 75% yield.
H¹ NMR ( δ ppm): 1,918(dd, ³*J*_{HF} =23,218 Hz, ³*J*_{HH} = 6,436 Hz, 3H, Me) ; 5,84 (dq, ²*J*_{HF} = 47,99 Hz, ³*J*_{HH} = 6,511 Hz, 1H, CHF) ; 7,65 (q, ³*J*_{HH} = 7,175 Hz, 2H, 2CH aro); 8,05 (d, ³*J*_{HH} = 8,064 Hz, 1H,CH aro); 8,27 (d, ³*J*_{HH} = 7,654 Hz, 1H,CH aro).

### Example 4a

### 2-(1-Fluoroethylsulfonyl)benzothiazole via fluorination with Selecftluor

To a solution of 2-(ethylsulfonyl)benzothiazole (21.3 g) in methylene chloride was added (60 g ) of FTEDA, portionwise. The mixture was stirred overnight, at room temperature, and then washed with water. The solvent was removed under reduced pressure and the crude product was purified by silica gel chromatography. Yield 65%.

### Example 5

### Olefination - general procedure :

### Method A (using potassium tert.-butylate as base):

To a solution of 2-(1-fluoroethylsulfonyl)benzothiazole (1eq) and the appropriate carbonyl compound (1.1 eq) in a solution of potassium tert.-butylate (1.1 eq or 2.2 eq.) in tetrahydrofuran was added tetrahydrofuran at certain temperature. The resulting solution was stirred definite time and then quenched by addition of aqueous ammonium chloride. The aqueous layer was extracted with methylene chloride and the extract washed with aqueous sodium hydroxide, water and dried over magnesium sulfate. After solvent evoparation, the crude product was purified by silica gel chromatography.

### Method B - using sodium bis(trimethylsilyl)amide as base):

To a solution of 2-(1-fluoroethylsulfonyl)benzothiazole (1 eq) and appropriate carbonyl compound (1.1 eq) in tetrahydrofuran, a solution of sodium bis(trimethylsilyl)amide (1.1 eq or 2.2 eq.) in THF was added slowly at -78°C. The mixture was stirred for 2h at -78°C and allowed to warm up to room temperature over 1 hour and then quenched by addition of aqueous ammonium chloride. The product was obtained by work-up in a similar manner to that described under Method A

### Example 6

in a similar manner to one of the methods of Example 5 the following compounds were obtained, from the appropriate carbonyl compound and 2-(1-fluoroethylsulfonyl)benzothiazole (sulfone).

### a) 1-(2-fluoro-1-propenyl)-4-nitrobenzene

This was obtained by method A from equimolar amounts of p-nitrobenzaldehyde and sulfone. Yield 67%.
(*Z*/*E* = 4/6).
H¹ NMR ( δ ppm): 2.14 (d, 3H, ³*J*_{HF} 17, H₃ (*Z*)) ; 2.17 (d, 3H, ³*J*_{HF} 18.1, H₃ (*E*)) ; 5.60 (d, 1 H, ³*J*_{HF} 37.4, H₁ (*Z*)) ; 6.25 (d, 1H, ³*J*_{HF} 20.7, H₁, (*E*)); 7.35 (d, 2H, ³*J*_{HH} 9, Ph (*Z*)) ; 7.58 (d, 2H, ³*J*_{HH} 8.9, Ph (*E*)) ; 8.15 (d, 2H, ³*J*_{HH} 9, Ph (*Z*)) ; 8.20 (d, 2H, ³*J*_{HH} 8.9, Ph (*E*)).
¹⁹F NMR (δ ppm): -82.2 (dq, ³*J*_{HF} 20.7, ³*J*_{HF} 18.2, *E* isomer); -88.2 (dq, ³*J*_{HF} 37.3, ³*J*_{HF} 17, *Z* isomer).

### b) (5-fluoro-1,4-pentadienyl)benzene

This was obtained by method A from from trans-cinnamaldehyde and sulfone.
Yield 71%. (*ZIE =* 4/6).
H¹ NMR ( δ ppm): 2.0 (d, 3H, ³*J*_{HF} 16.6, H₁ (*Z*)) ; 2.1 (d, 3H, ³*J*_{HF} 17.3, H₁ (*E*)) ; 5.4 (dd, 1H, ³*J*_{HH} 10.2, ³*J*_{HF} 34.7, H₃ (*Z*)), 5.92 (dd, 1H, ³*J*_{HH} 11, ³*J*_{HF} 19.5, H₃ (*E*)) ; 6.42 (dd, 1H, ³*J*_{HH} 10.2, ³*J*_{HH} 15.6, H₄ (*Z*)); 6.65 (dd, 1H, ³*J*_{HH} 11, ³*J*_{HH} 15.5, H₄ (*E*)) ; 6.96 (d, 1H, ³*J*_{HH} 15.7, H₅ (*z*)) ; 7.05 (d, 1 H, ³*J*_{HH} 15.5, H₅ (*E*)); 7.10-7.45 (m, 10H, Ph).
¹⁹F NMR ( δ ppm): -91.7 (dq, ³*J*_{HF} 17.7, ³*J*_{HF} 17.7, *E* isomer) ; -97.0 (dq, ³*J*_{HF} 34.3, ³*J*_{HF} 16.6, Z isomer).

### c) 4-(2-fluoro-1-propenyl) 2-methoxyphenol.

This was obtained by method A from 4-hydroxy-3-methoxybenzaldehyde (vanillin) and sulfone Yield 70%. (*Z*/*E* = 1/1).
H¹ NMR ( δ ppm): 2.05 (d, 3H, ³*J*_{HF} 16.9, H_{3'} (*E*)); 2.10 (d, 3H, ³*J*_{HF} 18.0, H_{3'} (*Z*)) ; 3.90 (s, 3H, OCH₃) ; 5.65 (br s, 1H, OH) ; 5.90 (d, 1H, ³*J*_{HF} 39, H_{1'} (*Z*)) ; 6.15 (d, 1H, ³*J*_{HF} 21.5, H_{1'} (*E*)) ; 6.65-7.15 (m, 3H, Ph).
¹⁹F NMR( δ ppm): -91.7 (dq, ³*J*_{HF} 21.6, ³*J*_{HF} 18 *E* isomer) ; -97.9 (dq, ³*J*_{HF} 39.2, ³*J*_{HF} 16.9 *Z* isomer).

### d) 2-fluoro-5,9-dimethyl-2,4,8-decatriene (E/Z : 75/25)

This was obtained by method B from 3,7-dimethyl-2,6-octadienal (citral) and sulfone. Yield 58%. (*Z*/*E* )F= 7/3).
1 H NMR( δ ppm): 1.53 (br s, 6H, H₁₀); 1.61 (br s, 9H, H_{9'}, H_{5'} (*E*)) ; 1.72 (br s, 3H, H₅, (*Z*)) ; 1.87 (d, 3H, ³*J*_{HF} 16.4, H₁ (*Z*)) ; 1.92 (d, 3H, ³*J*_{HF} 17.5, H₁ (*E*)) ; 2.05 (br s, 8H, H₆, H₇) ; 5.05 (br s, 2H, H₈) ; 5.28 (dd, 1 H, ³*J*_{HH} 11, ³*J*_{HF} 36, H₃ (*Z*)) ; 5.55 (d, 1H, ³*J*_{HH} 11.5, H₄ (*E*)); 5.83 (dd, 1H, ³*J*_{HH} 11.5, ³*J*_{HF} 21, H₃ (*E*)) 5.97 (d, 1H, ³*J*_{HH} 11, H₄ (*Z*)).
¹⁹F NMR (δ ppm): -92.25 (dq, ³*J*_{HF} 18, *(E, trans*)); -95.65 (dq, ³*J*_{HF} 18, *(E, cis))* ; -100.7 (dq, ³*J*_{HF} 33, ³*J*_{HF} 16, *(Z, trans*)) ; -101.1 (dq, ³*J*_{HF} 33, ³*J*_{HF} 16, *(Z, cis*)).

### e) 2-fluoro-1,1-diphenylpropene

This was obtained according to method B from benzophenone and sulfone.
Yield 52%.
H¹ NMR (δ ppm): 2.10 (d, ³*J*_{HF} 17.95 Hz, 3H, C₁) ; 7.15-7.95 (m, 10H, Ph).
¹⁹F NMR( δ ppm): -97.1 (q, ³*J*_{HF} 18 Hz).

### f) 4-tert.-butyl-(1-fluoroethylidene)cyclohexane

The compound was obtained according the method B from 4-tert.-butylcyclohexanone and sulfone Yield 61%.
H¹ NMR ( δ ppm): 0.71 (s, 9H, *t*Bu) ; 0.84 (m, 2H) ; 1.62 (m, 4H) ; 1.70 (d, 34, ³*J*_{HF} 17.8) ; 2.15 (m, 2H) ; 2.75 (m, 1H).
¹⁹F NMR (δ ppm): -107.6 (br q, ³*J*_{HF} 18 Hz).

### g) Methyl(1R-cis)-3-[(E)-2-fluoro-1-propenyl]-2,2-dimethylcyclopropane-1-carboxylate and Methyl(1R,cis)-3-[(Z)-2-fluoro-1-propenyl]-2,2-dimethylcyclopropane-1-carboxylate

A solution of methyl (1R,cis)-2-formyl-3,3-dimethylcyclopropane-1-carboxylate (biocartol methyl ester) (7.63 g, 0.049 mol) and sulfone (10 g, 0.04 mol) in tetrahydrofuran (100 ml) was cooled to -17°C and a solution of potassium tert.-butylate (8.96 g, 0.08 mol) in 50 ml tetrahydrofuran was slowly added at this temperature. The reaction mixture was slowly warmed to 0°C over 3h. The mixture was diluted with methylene chloride and washed with IN aqueous sodium hydroxide. The solution was dried over magnesium sulfate, evaporated under reduced pressure and the residue purified by flash column chromatography on silica gel (eluent: diethyl ether/petroleum ether: 95/5). Yield (5.98 g ; 80%) (ratio E/Z 51/49).
H¹ NMR(δ ppm): 1,190-1,213 (m, 12H, CH₃ (*E,Z*)) ; 1,625-1,70 (m, 3H, H₃,H₁(E), H₁(Z)) ; 1,91 (d, ³*J*_{HF} = 16,8 Hz, 3H, CH₃ (*Z*)) ; 1,92 (d, ³*J*_{HF} = 17,3 Hz, 3H, CH₃ (E)) ; 2,12 (t, ³*J*_{HH} = 9,3 Hz, 1H, H₃ (Z)) ; 3,60 (s, 6H, CO₂Me (*Z,E*)) ; 4,91 (dd, ³*J*_{HF} = 36,7 Hz, ³*J*_{HH} = 9,6 Hz, 1H, CH (Z)) ; 5,35 (dd, ³*J*_{HF} = 20,7 Hz, ³*J*_{HH} = 8,2 Hz, 1 H, CH (*E*)).
¹⁹F NMR( δ ppm):-92.5 (dq, ³*J*_{HF} 20.1, ³*J*_{HF} 17.4, *E* isomer) ; -103.2 (dq, ³*J*_{HF} 16.9, ³*J*_{HF} 37.2, Z isomer).
¹³C NMR ( δ ppm): 14.1 (d, ²*J*_{CF} 32.6, CH₃ (*E*)) ; 14.5, 14.6 (s, CH₃) ; 17.9 (d, ²*J*_{CF} 30.1, CH₃ (*Z*)); 28.2, 28.3 (s, C₁,); 28.5, 28.6 (s, CH₃) ; 29.5 (d, ³*J*_{CF} 12.6, C₃ (*Z*)) ; 30.4 (d, ³*J*_{CF} 9.6, C₃(*E*)) ; 51.0, 51.1 (s, OCH₃) ; 99.5 (d, ²*J*_{CF} 11, CH (*E*)); 100.6 (d, ²*J*_{CF} 28, CH (*Z*)) ; 158.2 (d, ¹*J*_{CF} 249.7, CF), 171.1 (s, CO), 171.5 (s, CO).

In a similar manner, starting from (1R,cis)-2-formyl-3,3-dimethyl-cyclopropane-1-carboxylic acid and using 3 equivalents of potassium tert.-butylate, (1R-cis)-3-[(E)-2-fluoro-1-propenyl)-2,2-dimethyl-cyclopropane-1-carboxylic acid, was obtained as an oil, in 82% yield.
Z/E 1:1.
¹H-NMR (δ ppm): 1,22 (d, 6H), 1.7 (t,2H); 1.92 (d, 3H), 5.4 (dd, 1H) ;
¹⁹F NMR (δ ppm): -93.3 (d.qw, J=21 ; 19 Hz) ppm
(1R-cis)-3-[(Z)-2-fluoro-1-propenyl)-2,2-dimethylcyclopropane-1-carboxylic acid.
¹H-NMR (δ ppm): 1,24 (d, 6H), 1.7 (m,1H);1.96 (d,3H); 2.2 (m,1H), 4.86(dd, 1H);
¹⁹F NMR ( δ ppm): -103.7 (d.qw, J=39; 18 Hz) ppm. (δ ppm): α

### h) 2-fluoropropene.

The compound was obtained by method B from formaldehyde and sulfone. Yield 48%. B.P.-23°C.

### i) (2-fluoro-1-propenyl)cyclohexane

The compound was obtained by method B from cyclohexanecarboxaldehyde and sulfone.
Yield 82%. Boiling point 85-87°C/35 mbar, cis/trans 1:1.
NMR ¹⁹F -99.10 (d, kw) cis: 104.32 (d,kw) trans.

### j) (2-fluoro-1-propenyl)benzene

The compound was obtained by method A from benzaldehyde and sulfone. Yield 85%

### k) 2-fluoro-2-hexene

The compound was obtained by method B from butanal and sulfone,
Yield 67%

## Claims

1. A process for the preparation of compounds of formula I 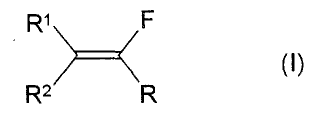 in which
R is hydrogen, optionally substituted alkyl, optionally substituted alkenyl optionally substituted cycloalkyl, optionally substituted phenyl or optionally substituted heterocyclyl, and
R¹ and R², which may be the same or different, are hydrogen, an organic radical or together with the ring to which they are attached, form an optionally substituted ring, which may contain one or more hetero atoms,
which comprises reacting a compound of formula II 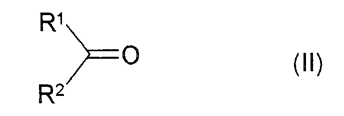 with a compound of formula III 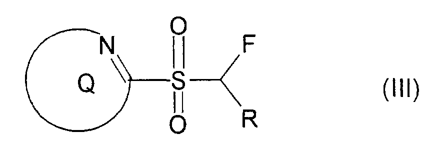 in which the ring Q is an optionally substituted heteroaromatic ring, which optionally comprises one or more further hetero atoms and to which is optionally fused an optionally substituted ring.

2. A process according to claim 1, in which the ring Q is benzothiazolyl.

3. A process according to claim 1 or 2, in which R is methyl.

4. A process according to any one of the preceding claims, in which R¹ is hydrogen.

5. A process according to any one of the preceding claims, in which R² is 3-carboxy-2,2-dimethylcyclopropyl.

6. A process according to claim 5 which comprises the further step of reacting the compound of formula I so obtained, or a functional derivative thereof, in which R is methyl, R¹ is hydrogen and R² is 3-carboxy-2,2-dimethylcyclopropyl, with an alcohol, ZOH, or a functional derivative thereof, where Z is the residue of a pyrethroid alcohol, to give a pyrethroid of formula X

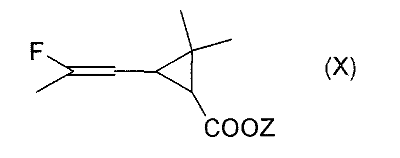

7. Compounds of formula I when obtained by a process claimed in any one of claims 1 to 5.

8. Compounds of formula X when obtained by a process claimed in claim 6.

9. Compounds of formula III 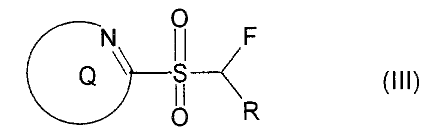 in which the ring Q and R are as defined in any one of claims 1 to 3.

10. A compound according to claim 9 which is 2-(1-fluoroethylthio)-benzothiazole.
